(19) Europäisches Patentamt
European Patent Office
Office européen des brevets

(11) Veröffentlichungsnummer: **0 160 804**
**B1**

(12) **EUROPÄISCHE PATENTSCHRIFT**

(45) Veröffentlichungstag der Patentschrift: **08.06.88**

(51) Int. Cl.⁴: **C 05 G 3/08,** C 07 D 231/12

(21) Anmeldenummer: **85102543.7**

(22) Anmeldetag: **06.03.85**

(54) **Nitrifikationsinhibierende 1-Hydroxypyrazol-Derivate.**

(30) Priorität: **14.03.84 DE 3409317**

(43) Veröffentlichungstag der Anmeldung: **13.11.85 Patentblatt 85/46**

(45) Bekanntmachung des Hinweises auf die Patenterteilung: **08.06.88 Patentblatt 88/23**

(84) Benannte Vertragsstaaten: **AT BE CH DE FR GB IT LI NL**

(56) Entgegenhaltungen:
**EP-A-0 087 615**
**GB-A-1 592 516**
**US-A-3 635 690**

**CHEMICAL ABSTRACTS, Band 91, 1979, Seite 673, Nr. 158074r, Columbus, Ohio, US; K. HORIKI et al.: "Amino acids, peptides, and related problems. Part 7. Neighboring group participation in peptide synthesis by the use of arylsulfonates of N-hydroxyazoles and related compounds - tautomeric effect" & HETEROCYCLES 1978, 10, 185-98**

(73) Patentinhaber: **BASF Aktiengesellschaft, Carl-Bosch- Strasse 38, D-6700 Ludwigshafen (DE)**

(72) Erfinder: **Rieber, Norbert, Dr., Liebfrauenstrasse 1c, D-6800 Mannheim (DE)**
Erfinder: **Boehm, Heinrich, Dr., Keplerweg 2, D-6708 Neuhofen (DE)**
Erfinder: **Pommer, Ernst- Heinrich, Dr., Berliner Platz 7, D-6703 Limburgerhof (DE)**
Erfinder: **Dressel, Juergen, Dr. Dipl.- Landwirt, 18 Medenheimer Strasse, D-6708 Neuhofen (DE)**

EP 0 160 804 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft 1-Hydroxipyrazole und deren Umsetzungsprodukte, Verfahren zu ihrer Herstellung und diese enthaltende Mittel zur Nitrifikationsinhibierung von Ammonium-Stickstoff.

Gebundener Stickstoff für die Pflanzenernährung kann im Boden in Form von Ammoniumverbindungen und von Nitraten vorliegen.

Ammoniumstickstoff wird durch Bakterien aus den Gattungen Nitrosomonas und Nitrobacter über Nitritstickstoff zu Nitratstickstoff oxidiert. Das Ausmaß der Nitrifikation ist abhängig von der Bodenart, dem pH-Wert des Bodens, der Bodenfeuchtigkeit und der biologischen Aktivität des Bodens. Da der in Form von Nitrat gebundene Stickstoff leichter als der in Ammoniumionen gebundene ausgewaschen wird und damit für die Pflanzenernährung nicht mehr verfügbar ist, außerdem die unerwünschte Nitrat-Anreicherung im Grundwasser fördert, kommt der Nitrifikationshemmung besondere Bedeutung zu. Diese Hemmung besteht nach allgemeiner Ansicht in einer selektiven Hemmung des Wachstums der vorgenannten Bakterienstämme.

Bekannt ist die Verwendung von N-substituierten Pyrazolen zur Nitrifikationsinhibierung (US 3 494 757 und 3 635 690, DE-OS 2 745 833 und GB 1 592 516).

Außerdem wird 2-Chlor-6-trichlormethylpyridin (Nitrapyrin) oder 3,5-Dimethyltetrahydro-1,3,5-thiadiazin-2-thion (Dazomet) zur Nitrifikationsinhibierung empfohlen.

Die bekannten Wirkstoffe genügen jedoch nicht allen Ansprüchen hinsichtlich Wirksamkeit, Wirkungsdauer, Wirtschaftlichkeit, Unschädlichkeit, anwendungstechnischer Eigenschaften wie Wasserlöslichkeit, Dispergierbarkeit, Dampfdruck usw. Stoffe wie Dazomet wirken auch sehr unspezifisch und greifen auch Bodenbakterien an, die nicht geschädigt werden sollen.

Die Aufgabe, Nitrifikationsinhibitoren zu schaffen, die die vorstehenden Nachteile nicht oder in geringerem Maße als die bekannten Mittel dieser Art aufweisen, wird gelöst durch 1-Hydroxipyrazolderivate der Formel (I)

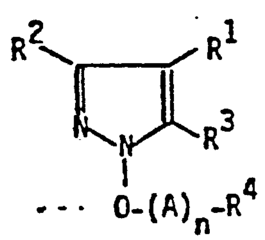

(I)

Hierbei bedeuten:

A den Rest $-\overset{O}{\overset{\|}{C}}-$, $-\overset{O}{\overset{\|}{C}}-O-$, $-\overset{O}{\overset{\|}{C}}-\overset{H}{N}-$, $-SO-$, $-SO_2-$ oder $-SO_2-\overset{R^4}{N}-$,

n 0 und 1,

$R^1$, $R^2$ und $R^3$ unabhängig voneinander Wasserstoff, Chlor, Brom oder Iod,

$R^4$ Wasserstoff, einen verzweigten oder unverzweigten, offenkettigen oder einen cyclischen Alkyl-, Alkenyl- oder Alkinylrest oder einen Aryl- bzw. Aralkylrest, wobei die substitutionsfähigen Reste $R^4$ auch ein- oder mehrfach durch Halogen, Halogenalkyl, Halogenalkoxi, Alkyl, Alkyloxi, Alkoxicarbonyl, -OH, =O, -COOH bzw. Salze davon, $-NO_2$ oder -CN substituiert sein können.

Stoffe der Formel I, bei denen $R^4$ nicht Wasserstoff ist, wenn $n = 0$ ist, sind neue Stoffe ausgenommem die Verbindung N-Hydroxypyrazol-4-chlorbenzolsulfonat (Chem. Abstracts, Bd. 91, 1979, Seite 673, Nr. 158074r). Sie können aus den - teilweise ebenfalls nitrifikationshemmenden - entsprechenden 1-Hydroxipyrazolen gewonnen werden. Diese können z. B. nach den Vorschriften der DE Offenlegungsschriften 30 31 385 und 32 05 456 bzw. der EP-Patentveröffentlichung 0 087 615 erhalten werden. Beispielsweise sin dort die folgende 1-Hydroxypyrazole ($n = 0$, $R^4 = H$) beschrieben:

| Beispiel | $R^1$ | $R^2$ | $R^3$ | Herstellung nach |
|---|---|---|---|---|
| 162 | H | H | H | DE-OS 30 31 385, Beispiel 1 |
| 163 | Cl | H | H | DE-OS 32 05 456, Tabelle |
| 164 | Br | H | H | " , Beispiel 1 |
| 165 | I | H | H | " , Beispiel 3 |
| 166 | Br | Br | H | " , Tabelle |
| 167 | Br | Br | Br | " , Tabelle |
| 168 | Cl | Cl | Cl | " , Tabelle |
| 169 | I | I | I | " , Beispiel 2 |
| 170 | Cl | Br | Br | " , Beispiel 4 |
| 171 | Cl | I | I | " , Tabelle |

Durch Umsetzung von 1-Hydroxipyrazolen mit aliphatischen oder aromatischen Halogenverbindungen lassen sich auf übliche Art und Weise unter Abspaltung von Halogenwasserstoff aliphatische und aromatische 1-Pyrazolylverbindungen herstellen. Hierzu erhitzt man die Reaktionsteilnehmer vorzugsweise in einem Lösungsmittel und in Anwesenheit einer Base, z. B. eines tertiären Amins, Alkalicarbonats oder von Natriummethylat bzw. -hydrid. Manchmal setzt die Umsetzung bereits bei Raumtemperatur, gelegentlich aber auch erst bei bis zu 18°C ein. Die Herstellung der 1-pyrazolylcarbonsäure-, -kohlensäure- und -sulfonsäureester, die sich aus Formel I ergeben, gelingt mit allgemein üblichen Methoden durch Umsetzung von 1-Hydroxipyrazolen mit Carbonsäurehalogeniden, Sulfonsäurehalogeniden oder Chlorkohlensäureestern. In Gegenwart von Basen verläuft diese Umsetzung oft schon bei -30°C bis zu 80°C.

1-Pyrazolylcarbaminsäureester der Formel I erhält man mit allgemein bekannten Methoden durch Umsetzung von 1-Hydroxipyrazolen mit aliphatischen oder aromatischen Isocyanaten durch einfaches Vermischen der Komponenten, im allgemeinen oberhalb von 0°C, in einem Lösungsmittel.

Iodpropargylether von 1-Hydroxipyrazolen werden z. B. aus entsprechenden Propargylethern der 1-Hydroxipyrazole durch Umsetzung mit Iod unter Iodwasserstoffabspaltung bei 20 bis 80°C erhalten. Die Gegenwart einer Base ist von Vorteil.

1-Pyrazolyloxicarbonsäuren lassen sich durch Verseifen der 1-Pyrazolyloxicarbonsäurealkylester (gemischten Pyrazolyl-alkyl-kohlensäureester) nach allgemein üblichen Methoden herstellen; bei alkalischer Hydrolyse mit wäßriger Natronlauge verläuft die Umsetzung im allgemeinen unterhalb von 80°C; dabei wird nur der Alkylrest verseift.

Die in den nachfolgenden Tabellen angegebenen erfindungsgemäßen Stoffe wurden entsprechend den Angaben der jeweils vorausstehenden ausführlichen Beispiele hergestellt und ihre Struktur mit üblichen Mitteln gesichert, soweit wenigstens eine physikalische Eigenschaft angegeben ist. Die ohne physikalische Daten aufgeführten Verbindungen können leicht unter entsprechender Abwandlung der angegebenen Herstellvorschriften erhalten werden.

**Beispiel 1**

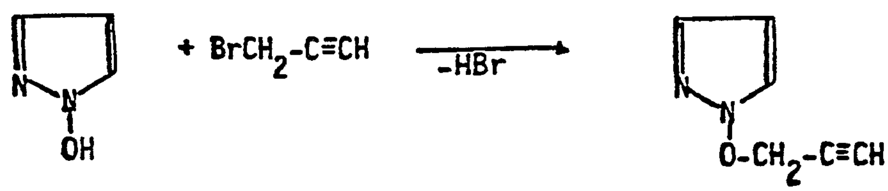


Zu je 15 g 1-Hydroxipyrazol und Natriumcarbonat in 200 g Acetonitril gibt man unter Rühren bei 25°C 21,3 g Propargylbromid. Nach 12 Stunden Rühren wird abgesaugt, der Rückstand mit 100 g Acetonitril gewaschen und die vereinigten Flüssigkeiten im Rotationsverdampfer bei 40°C und 20 mbar eingeengt. Der hierbei erhaltene Rückstand wird in 200 g Dichlormethan gelöst und 3 mal mit insgesamt 200 g gesättigter wäßriger Natriumhydrogencarbonatlösung extrahiert, mit Magnesiumsulfat getrocknet und eingeengt. Man erhält 15 g (70 % d.Th.) 1-Pyrazolylpropargylether als Öl ($H^1$-NMR, δ in ppm: 2.6, t, 1 H; 4.9, d, 2 H; 6.2, m, 1 H; 7.4, m, 2 H). Dieser Stoff ist in der nachstehenden Tabelle als Beispiel 10 enthalten.

**Beispiel 2**

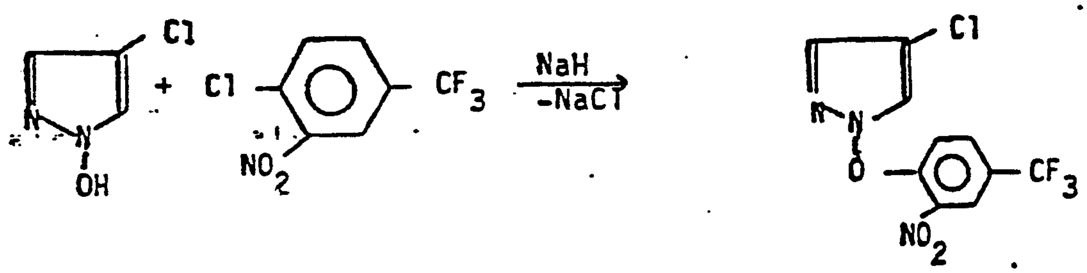


20 g 4-Chlorhydroxipyrazol, 6 g NaH und 30 g 1-Chlor-2-nitro-4-trifluormethylbenzol werden 25 Stunden bei 80°C gerührt. Danach wird auf 25°C abgekühlt, vom Ungelösten abfiltriert, der Rückstand mit 100 g Acetonitril gewaschen und die Lösung im Rotationsverdampfer eingeengt (40°C, 25 mbar). Den Rückstand löst man in 250 g Dichlormethan, extrahiert 3 mal mit insgesamt 200 g 1 %-iger wäßriger Natronlauge, danach 3 mal mit insgesamt 200 g Wasser und engt die organische Phase nach Trocknen mit Magnesiumsulfat ein. Man erhält 25 g (60 % d.Th.) 1-(2-Nitro-4-trifluormethyl)-phenoxi-4-chlorpyrazol als Öl ($H^1$-NMR, δ in ppm: 6.8, d, 1 H; 7.3, s, 1 H; 7.6, s, 1 H; 7.7, d, 1 H; 8.2, bs, 1 H). Dieser Stoff ist in der nachstehenden Tabelle als Beispiel 49 enthalten.

In der nachstehenden Tabelle bezieht sich der Kochpunkt ohne Index auf atmosphärischen Druck; die Indices sind in mbar zu lesen.

**Tabelle 1**

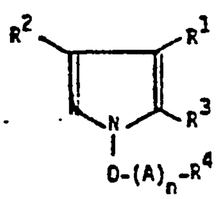

n = 0

| Beispiel | $R^1$ | $R^2$ | $R^3$ | $R^4$ | Fp. [°C] |
|---|---|---|---|---|---|
| 3 | H | H | H | $CH_3$ | Kp 132 |
| 4 | Cl | " | " | " | Öl |
| 5 | H | " | " | 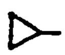 | $Kp_{20}$ 86 |
| 6 | " | " | " | $(CH_3)_2CH-$ | $Kp_{18}$ 75 |
| 7 | " | " | " | $(CH_3)_3C-$ | $Kp_{20}$ 90 |
| 8 | " | " | " | $CH_2=CH-CH_2$ | Kp 168 |
| 9 | " | " | " | $Cl_2C=CCl-CH_2-$ | |
| 10 | Cl | " | " | $NC-CH_2-$ | Öl |
| 11 | I | " | " | $HC\equiv C-CH_2-$ | " |
| 12 | Br | " | " | " | " |
| 13 | " | Br | " | " | 67 |
| 14 | Cl | H | " | " | Öl |
| 15 | " | Br | Br | " | " |
| 16 | Br | Br | Br | " | " |
| 17 | Cl | Cl | Cl | " | " |
| 18 | H | H | H | $C_3H_7-CH=CH-CH_2-$ | " |
| 19 | " | " | " | $C_2H_5-C\equiv C-CH_2-$ | " |
| 20 | " | " | " | $C_3H_7-C\equiv C-CH_2-$ | " |

| | | | | | |
|---|---|---|---|---|---|
| 21 | ″ | ″ | ″ | $CH_3$-$(CH_2)_{10}$-$CH_2$- | ″ |
| 22 | Cl | ″ | ″ | ″ | ″ |
| 23 | H | ″ | ″ | 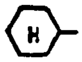 | |
| 24 | ″ | ″ | ″ | $CH_3OOC$-$CH_2$- | Öl |
| 25 | Cl | ″ | ″ | ″ | ″ |
| 26 | Br | ″ | ″ | ″ | ″ |
| 27 | ″ | Br | ″ | ″ | 101 |
| 28 | ″ | ″ | Br | ″ | 72 |
| 29 | H | H | H | $CH_3OOC$-$(CH_2)_2$- | Öl |
| 30 | ″ | ″ | ″ | $CH_3OOC$-$(CH_2)_3$- | ″ |
| 31 | Cl | ″ | ″ | ″ | ″ |
| 32 | H | ″ | ″ | $CH_3OOC$-$(CH_2)_4$- | ″ |
| 33 | ″ | ″ | ″ | $CH_3OOC$-$CH(CH_3)$- | ″ |
| 34 | ″ | ″ | ″ | $ICH_2$-$(CH_2)_2$- | ″ |
| 35 | ″ | ″ | ″ | $CH_6H_5$-$CH_2$- | $Kp_{0,5}$ 146 |
| 36 | ″ | ″ | ″ | 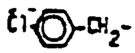 | 44 |
| 37 | ″ | ″ | ″ | $(CH_3)_3C$-CO-$CH_2$- | 33 |
| 38 | Cl | ″ | ″ | ″ | 68 |
| 39 | H | ″ | ″ | 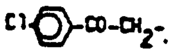 | 124 |
| 40 | ″ | ″ | ″ | 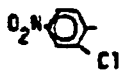 | 69 |
| 41 | Cl | ″ | ″ | 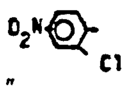 | Öl |
| 42 | ″ | Br | Br | ″ | ″ |
| 43 | H | H | H | 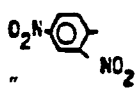 | 116 |
| 44 | Cl | ″ | ″ | ″ | Öl |
| 45 | Br | ″ | ″ | ″ | ″ |
| 46 | H | ″ | ″ | 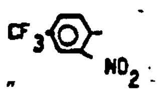 | 66 |
| 47 | I | ″ | ″ | ″ | 63 |
| 48 | Br | ″ | ″ | ″ | Öl |
| 49 | Cl | ″ | ″ | ″ | ″ |
| 50 | ″ | Br | Br | ″ | ″ |
| 51 | H | H | H | 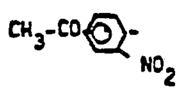 | ″ |

**Beispiel 53**

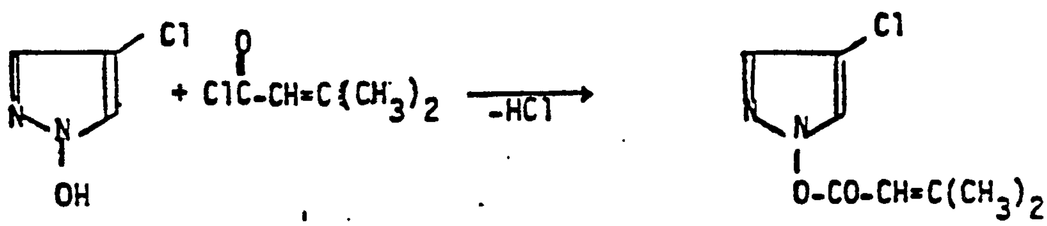


Zu 20 g 4-Chlor-1-hydroxipyrazol, 20 g Natriumcarbonat und 150 g Acetonitril gibt man bei 25°C unter Rühren 20 g Dimethylacrylsäurechlorid, gelöst in 100 g Acetonitril. Nach 12 Stunden Rühren bei 25°C wird das Festprodukt abgesaugt und mit 100 g Acetonitril gewaschen. Man engt die Lösung bei 40°C und 20 mbar ein,

nimmt den Rückstand in 200 g Dichlormethan auf und extrahiert 3 mal mit 200 g gesättigter wäßriger Natriumbicarbonatlösung. Die organische Phase wird mit Magnesiumsulfat getrocknet, eingeengt und der Rückstand mit 300 g Petrolether digeriert. Man erhält 30,5 g (90 % d.Th.) 1-(4-Chlor)-pyrazolyl-3,3-dimethylacrylat mit dem Fp. 71°C.

**Tabelle 2**

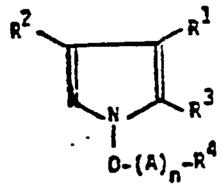

n = 1

| Beispiel | $R^1$ | $R^2$ | $R^3$ | A | $R^4$ | Fp. [°C] |
|---|---|---|---|---|---|---|
| 53 | H | H | H | CO | $CH_3$- | $Kp_{20}$ 95 |
| 54 | " | " | " | $SO_2$ | " | 34 |
| 55 | Cl | " | " | $CO_2$ | " | Öl |
| 56 | Br | " | " | CO | " | " |
| 57 | " | Br | " | $CO_2$ | " | " |
| 58 | " | " | Br | " | " | 65 |
| 59 | Cl | " | " | CO | " | 87 |
| 60 | " | " | " | " | $C_2H_5$- | 83 |
| 61 | " | " | " | " | $C_3H_7$- | 41 |
| 62 | H | H | H | $SO_2$ | $(CH_3)_2CH$- | Öl |
| 63 | " | " | " | $SO_2NH$ | " | 79 |
| 64 | Cl | " | " | " | " | 104 |
| 65 | Br | Br | " | " | " | 142 |
| 66 | H | H | " | $CO_2$ | " | Öl |
| 67 | Cl | " | " | " | " | " |
| 68 | Br | " | " | " | " | " |
| 69 | " | Br | " | " | " | " |
| 70 | " | " | Br | " | " | " |
| 71 | H | H | H | CO | 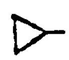 | 35 |
| 72 | " | " | " | " | 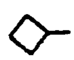 | Öl |
| 73 | " | " | " | " | 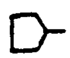 | " |
| 74 | " | " | " | " | 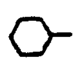 | " |
| 75 | " | " | " | $CO_2$ | " | " |
| 76 | " | " | " | CO | $(CH_3)_3C$- | " |
| 77 | Cl | " | " | " | " | " |
| 78 | H | " | " | " | $(CH_3)_2CH-CH_2$- | " |
| 79 | Cl | " | " | " | " | " |
| 80 | H | " | " | " | $(CH_3)_3C-CH_2$- | " |
| 81 | Cl | " | " | " | " | " |
| 82 | H | " | " | " | $CH_2=C(CH_3)$- | " |
| 83 | Cl | " | " | " | " | " |
| 84 | H | " | " | " | $CH_3-CH=C(CH_3)$- | " |
| 85 | Cl | " | " | " | " | " |
| 86 | H | " | " | " | $HC\equiv C-CH_2$- | $Kp_1$ 50 |
| 87 | " | " | " | " | $CH_3-(CH_2)_7$- | 30 |
| 88 | " | " | " | " | $CH_3-CH=CH$- | 58 |
| 89 | Cl | " | " | " | " | 34 |
| 90 | Br | " | " | " | " | 60 |
| 91 | " | Br | " | | " | 86 |
| 92 | " | H | " | " | $(CH_3)_2C=CH$- | 69 |
| 93 | H | " | " | " | " | 44 |
| 94 | Br | Br | " | " | " | 57 |

| | | | | | | |
|---|---|---|---|---|---|---|
| 95 | " | " | Br | " | " | 61 |
| 96 | Br | Br | Br | CO | $CH_3$-CH=CH- | 94 |
| 97 | Cl | " | " | " | $CH_3$-$(CH_2)_7$- | Öl |
| 98 | " | " | " | " | $CH_3$-$(CH_2)_{16}$- | 94 |
| 99 | " | " | " | " | $CH_3$-$(CH_2)_7$-CH=CH-$(CH_2)_7$- | Öl |
| 100 | " | H | H | " | $CH_2$=CH-$(CH_2)_8$- | " |
| 101 | H | " | " | " | " | " |
| 102 | " | " | " | $SO_2$ | $C_6H_5$-CH=CH- | " |
| 103 | " | " | " | CO | " | 85 |
| 104 | Cl | " | " | $SO_2$ | " | Öl |
| 105 | " | " | " | CO | " | 80 |
| 106 | H | " | " | $CO_2$ | $(CH_3)_3C$- 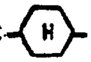 | 55 |
| 107 | " | " | " | CO | $C_6H_5$-CH(Cl)- | 39 |
| 108 | " | " | " | " | 2,4-DiCl-$C_6H_3$-O-$CH_2$- | 85 |
| 109 | " | " | " | $CO_2$ | $ClCH_2$-$CH_2$- | Öl |
| 110 | " | " | " | " | $CH_3O$-$CH_2$-$CH_2$- | " |
| 111 | " | " | " | CO | $CH_3$-CH(Cl)- | " |
| 112 | " | " | " | " | Cl-$CH_2$-$CH_2$- | " |
| 113 | " | " | " | " | $CH_3$-CH(Br)- | 64 |
| 114 | " | " | " | $CO_2$ | $ClH_2$-$CH_2$-$CH_2$- | Öl |
| 115 | Cl | " | " | $SO_2$ | $C_6H_5$ | " |
| 116 | Br | " | " | " | " | 50 |
| 117 | H | " | " | " | 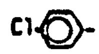 | 73 |
| 118 | Cl | " | " | " | " | Öl |
| 119 | H | " | " | CO | " | 75 |
| 120 | " | " | " | $CO_2$ | $C_6H_5$- | 65 |
| 121 | Cl | Br | Br | CO | " | 86 |
| 122 | Br | H | H | $SO_2$ | 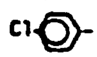 | 92 |
| 123 | Cl | Br | Br | CO | | 105 |
| 124 | H | H | H | $SO_2$ | 2,4,5-TriCl-$C_6H_2$- | 85 |
| 125 | Cl | " | " | " | " | 92 |
| 126 | Br | " | " | " | " | 103 |
| 127 | " | Br | " | " | " | 106 |
| 128 | " | " | " | " | 4-Cl-$C_6H_4$- | Öl |
| 129 | I | H | " | " | $C_6H_5$- | 62 |
| 130 | " | " | " | " | 4-Cl-$C_6H_4$- | 100 |
| 131 | Br | Br | Br | " | " | 142 |
| 132 | H | H | H | $SO_2$ | 4-$CH_3$-$C_6H_4$- | 45 |
| 133 | " | " | " | " | 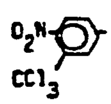 | 148 |
| 134 | Cl | " | " | " | " | 87 |

**Beispiel 135**

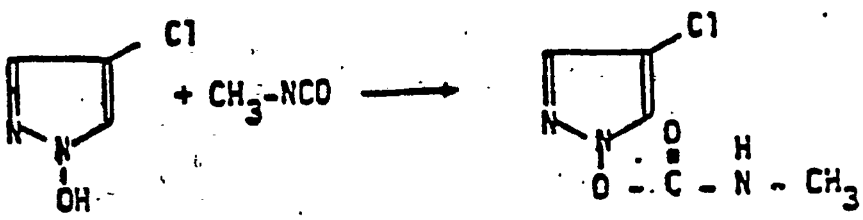

Zu 15 g 4-Chlor-1-hydroxipyrazol und 150 g Acetonitril gibt man bei 25°C unter Rühren 8 g Methylisocyanat, gelöst in 50 g Acetonitril. Nach 12 Stunden Rühren wird das Reaktionsgemissh eingeengt und der Rückstand 3 mal mit insgesamt 150 g Petrolether digeriert. Man erhält 17,8 g (80 % d.Th.) 1-(4-Chlor)pyrazolyl-N-methylcarbamat mit dem Fp. 125°C.

**Tabelle 3**

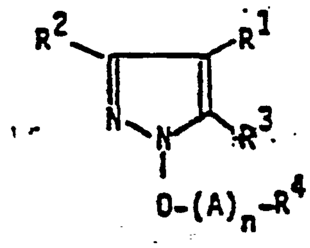

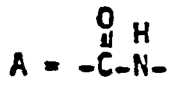

n = 1

| Beispiel | $R^1$ | $R^2$ | $R^3$ | $R^4$ | Fp. [°V] |
|---|---|---|---|---|---|
| 136 | H | H | H | $CH_3$- | 96 |
| 137 | I | H | H | $CH_3$- | 158 |
| 138 | Br | Br | H | $CH_3$- | 148 |
| 139 | Br | Br | Br | $CH_3$- | 156 |
| 140 | H | H | H | $C_4H_9$- | 59 |
| 141 | H | H | H | 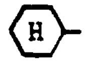 | 117 |
| 142 | H | H | H | 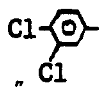 | 178 |
| 143 | Br | H | H | " | 225 |
| 144 | Br | Br | Br | " | 174 |

**Beispiel 145**

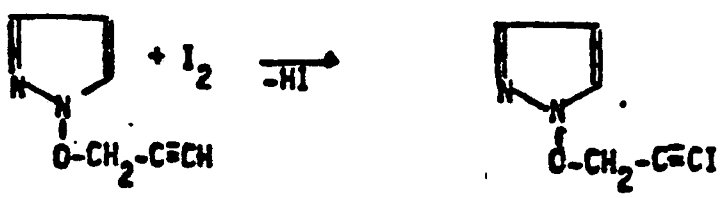


Zu 20 g 1-pyrazolylpropargylether, 20 g Natriumcarbonat in 400 g Acetonitril gibt man portionsweise bei 40° C unter Rühren 41,6 g Iod. Nach 8 Stunden Rühren bei 40° C wird vom Ungelösten abgesaugt und das Filtrat im Rotationsverdampfer bei 30° C und 20 mbar eingeengt. Der Rückstand wird in 200 g Dichlormethan gelöst und 2 mal mit 200 g 10 %-iger wäßriger Natriumthiosulfatlösung extrahiert. Die organische Phase wird mit Magnesiumsulfat getrocknet und eingeengt. Man erhält 40 g (99 % d.Th.) 1-pyrazolyl-iodpropargylether als Öl ($H^1$-NMR, δ in ppm: 5.1, s, 2 H; 6.2, m, 1 H; 7.4, m, 2 H).

**Tabelle 4**

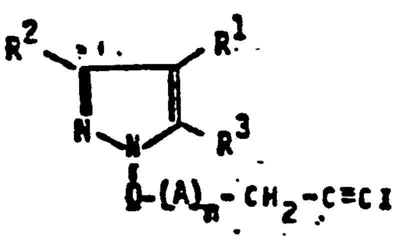

n = 0

$R^4 = -CH_2-C\equiv CI$

| Beispiel | $R^1$ | $R^2$ | $R^3$ | Fp. [°C] |
|---|---|---|---|---|
| 146 | Cl | H | H | Öl |
| 147 | Br | H | H | " |
| 148 | Br | Br | H | " |
| 149 | Br | Br | Br | " |
| 150 | Cl | Br | Br | " |
| 151 | I | H | H | " |
| 152 | Cl | Cl | Cl | " |

**Beispiel 159**

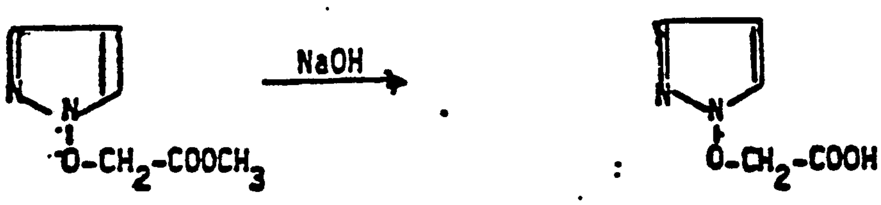


15,6 g 1-pyrazolyl-oxiessigsäuremethylester und 8 g 50 %-ige wäßrige Natronlauge werden 3 Stunden bei 50° C gerührt. Des Gemisch wird mit 10 %-iger Salzsäure auf pH 2 angesäuert und 2 mal mit 200 g Dichlormethan extrahiert. Die organische Phase trocknet man mit Magnesiumsulfat und engt eim. Man erhält 10,5 g (75 % d.Th.) 1-pyrazolyl-oxiessigsäure mit dem Fp. 99° C.

**Tabelle 5**

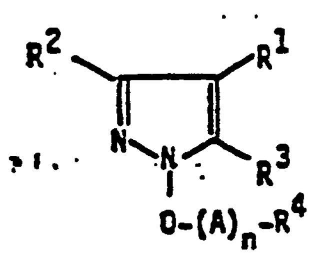

n = 0

| Beispiel | $R^1$ | $R^2$ | $R^3$ | $R^4$ | Fp. [° C] |
|---|---|---|---|---|---|
| 154 | Cl | H | H | HOOC-$CH_2$- | 104 |
| 155 | Br | H | H | " | 66 |
| 156 | Br | Br | Br | " | 84 |
| 157 | H | H | H | HOOC-$(CH_2)_2$- | 51 |
| 158 | H | H | H | HOOC-$(CH_2)_3$- | 48 |
| 159 | Cl | H | H | " | 73 |
| 160 | H | H | H | HOOC-$(CH_2)_4$- | 32 |
| 161 | H | H | H | HOOC-CH($CH_3$)- | 124 |

| Beispiel | δ[ppm] |
|---|---|
| 11 | 2.65,t, 1H; 4.9,d, 2H; 7.3,s, 1H; 7.7,s, 1H |
| 12 | 2.7,t, 1H; 4.9,d, 2H; 7.3,s, 1H; 7.5,s 1H |
| 14 | 2.65,t 1H; 4.9,D 2H; 7.25,s, 1H; 7.5,s, 1H |
| 15 | 2.7,t, 1H; 4.9,d, 2H |
| 16 | 2.65,t, 1H; 4.9,d, 2H |
| 17 | 2.65,t, 1H; 4.9,d, 2H |
| 18 | 0.7-2.2,m, 7H; 4.7,m, 2H; 5.7,m, 2H; 6.1,m, 1H, 7.3,d, 2H |
| 19 | 1.1,t, 3H; 2.2,m, 2H; 4.9,m, 2H; 6.2,m, 1H; 7.4,m, 2H |
| 20 | 0.9,t, 3H; 1.4,m, 2H; 2.2,m, 2H; 4.9,s, 2H; 6.2,m, 1H; 7.4,m, 2H |
| 21 | 0.9,m, 3H; 1.3,m, 20H; 4.3,m 2H; 6.2,t, 1H; 7.3,m, 2H |
| 22 | 0.9,m, 3H; 1.3,m, 20H; 4.3,m 2H; 7.2,s, 1H; 7.5,s, 1H |
| 24 | 3.8,s, 3H; 4.9,s, 2H; 6.2,t, 1H; 7.2,m, 1H; 7.5,m, 1H |
| 25 | 3.8,s, 3H; 4.9,s, 2H; 7.25,s, 1H; 7.5,s, 1H |
| 26 | 3.7,s, 3H; 4.8,s, 2H; 7.2,s, 1H; 7.6,s, 1H |
| 29 | 1.2,t, 3H; 2.7,t, 2H; 4.3,m, 4H; 6.2,m, 1H; 7.3,m, 2H |
| 30 | 2.4,m, 4H; 3.7,s 3H; 4.3,m, 2H; 6.1,t, 1H; 7.3,m, 2H |

| | |
|---|---|
| 31 | 2.4,m, 4H; 3.7,s, 3H; 4.3,m, 2H; 7.2,s 1H; 7.5,s, 1H |
| 32 | 2.0,m, 4H; 2.5,m, 2H; 3.7,s, 3H; 4.3,m, 2H; 6.2,m, 1H; 7.3,m, 2H |
| 33 | 1.5,d, 3H; 3.6,s, 3H; 5.0,mm 1H; 6.0,t, 1H; 7.25,m, 2H |
| 34 | 2.1,m, 2H; 3.3,t, 2H; 4.4,t, 2H; 6.1,t, 1H; 7.2,m, 2H |
| 41 | 6.6,d, 1H; 7.5,d, 2H; 8.1,m, 1H; 8.4,d, 1H |
| 42 | 6.6,d, 1H; 8.1,m, 1H; 8.4,d, 1H |
| 44 | 6.9,d, 1H; 7.6,d, 2H; 8.4,m, 1H; 8.9,d, 1H |
| 45 | 6.8,d, 1H; 7.5,d, 2H; 8.3,m, 1H; 8.8,d, 1H |
| 48 | 6.8,d, 1H; 7.35,s, 1H; 7.6-8.3,m, 3H |
| 50 | 6.6,d, 1H; 7.6,m, 1H; 8.1,d, 1H |
| 51 | 2.6,s, 3H; 6.4,m, 2H; 7.4,d, 1H; 7.6,d, 1H; 8.0,m, 1H; 8.5,d, 1H |
| 55 | 4.1,s, 3H; 7.4,d, 2H |
| 56 | 4.0,s, 3H; 7.35,d, 2H |
| 57 | 4.0,s, 3H; 7.4,s, 1H |
| 62 | 2.6,d, 6H; 3.9,m, 1H; 6.3,t, 1H; 7.4,m, 2H |
| 66 | 1.4,d, 6H; 5.05,m, 1H; 6.3,t, 1H; 7.4,m, 2H |
| 67 | 1.4,d, 6H; 5.1,m, 1H; 7.4,m, 2H |
| 68 | 1.4,d, 6H; 5.0,m, 1H; 7.4,d, 2H |
| 69 | 1.4,d, 6H; 5.05,m, 1H; 7.4,s, 1H |
| 70 | 1.4,d, 6H; 5.1,m, 1H |
| 72 | 2.2,m, 6H; 3.3,m, 1H; 6.3,t, 1H; 7.3,m, 2H |
| 73 | 1.3-2.3,m, 8H; 2.0,m, 1H; 6.3,m, 1H; 7.3,m, 2H |
| 74 | 1.2-3.0,m, 11H; 6.4,t, 1H; 7.4,m, 2H |
| 75 | 1.5,m, 10H; 4.7,m, 1H; 6.2,m, 1H; 7.3,m, 2H |
| 76 | 1.4,s, 9H; 6.35,t, 1H; 7.4,m, 2H |
| 77 | 1.4,s, 9H; 7.4,m, 2H |
| 78 | 1.0,d, 6H; 2.3,m, 3H; 6.3,t, 1H; 7.4,m, 2H |
| 79 | 1.1,d, 6H; 2.4,m, 3H; 7.4,d, 2H |
| 80 | 1.2,s, 9H; 2.5,s, 2H; 6.4,t, 1H; 7.4,m, 2H |
| 81 | 1.2,s, 9H; 2.5,s, 2H; 7.3,m, 2H |
| 82 | 2.1,d, 3H; 5.9,bs 1H; 6.4,M, 2H; 7.4,d 2H |
| 83 | 2.1,d, 3H; 5.9,bs, 1H; 6.5,bs, 1H |
| 84 | 1.9,d, 6H; 6.4,t, 1H; 7.3,m, 3H |
| 85 | 1.9,d, 6H; 7.4,m, 3H |
| 97 | 0.7-1.9,m, 15H; 2.5,m, 2H |
| 99 | 0.7-2.7,m, 31H; 5.3,m, 2H |
| 100 | 1.1-2.8,m, 16H; 4.8-5.3,m, 2H; 5.5-6.2,m, 1H; 7.4,m, 2H |
| 101 | 1.1-2.8,m, 16H; 4.8-5.3,m, 2H; 5.5-6.4,m, 2H; 7.4,m, 2H |
| 102 | 6.3,m, 1H; 6.65-7.7,m, 9H |
| 104 | 6.7-7.7,m, 9H |
| 109 | 3.7,t, 2H; 4.5,t, 2H; 6.2,t, 1H; 7.4,m, 2H |
| 110 | 3.4 s, 3H; 3.6,m, 2H; 4.5,m 2H; 6.3,t 1H; 7.4,m, 2H |
| 111 | 1.7,t 1H; 4.5,m 1H; 6.15,m, 1H; 7.3,m, 2H |
| 112 | 3.0,t, 2H; 3.8,t, 2H; 6.3,t, 1H; 7.3,d, 2H |
| 114 | 2.25,m, 2H; 2.8,t, 2H; 3.60,t, 2H; 6.3,t, 1H; 7.3,d, 2H |
| 115 | 7.2,s, 1H; 7.4-8.1,m, 6H |
| 118 | 7.1,s, 1H; 7.3-8.1,m, 5H |
| 128 | 7.5-8.0,m, 5H |
| 146 | 5.2,s, 2H; 7.4,m, 2H |
| 147 | 5.1,s, 2H; 7.35,m, 2H |
| 148 | 5.2,s, 2H; 7.3,m, 2H |
| 149 | 5.2,s, 2H |
| 150 | 5.15,s, 2H |
| 151 | 5.2,s, 2H |
| 152 | 5.2,s, 2H; 7.25,s, 1H; 7.6,s, 1H |

**Anwendungsbeispiel**

Zu 200 g eines nicht sterilisierten, dem Freiland entnommenen lehmigen Sandbodens, dessen Feuchtigkeitsgehalt auf 50 % der maximalen Wasserkapazität eingestellt war, wurden 220 mg Ammoniumsulfat gegeben und mit dem Boden gründlich vermischt. Danach wurden die Wirkstoffe, in 0,2 ml Aceton gelöst, in Mengen von jeweils 1 ppm, bezogen auf feuchten Sandboden, zugesetzt. Nach sorgfältiger Durchmischung

wurden die Bodenproben nach Verdunsten des Acetons in mit Aluminiumfolie zur Vermeidung von Wasserverlusten abgedeckten 1-Liter-Gläsern zusammen mit den Kontrollen ohne Wirkstoffzusatz über einen Zeitraum von 28 Tagen bei 21°C bebrütet (Nach diesem Zeitraum enthält eine Bodenprobe mit normaler Bodengare im allgemeinen keine nachweisbaren Mengen von Ammoniumstickstoff mehr).

Danach wurden jeweils 2,5 g der Bodenproben in 100-ml -Erlenmeyerkolben eingefüllt und 22,5 ml einer 0,1n Kaliumsulfat-Lösung zugesetzt. Nach 30minütigem Schütteln wurde abfiltriert und jeweils 2,5 ml der Bodenauszüge wurden mit 1625 ml destilliertem Wasser vermischt. Anschließend wurden zum Nachweis der im Bodenauszug noch vorhandenen Ammoniumionen 1,25 ml Neßler-Reagenz hinzugefügt und gründlich geschüttelt. Die Farbveränderungen wurden dann photometrisch bei einer Wellenlänge von 420 mm gemessen. Anhand von Standardkurven, die durch Messung von Lösungen mit bekannten Ammoniumsulfat-Gehalten ermittelt worden waren, wurden die noch in den Bodenproben vorhandenen Ammoniumsulfat-Mengen bestimmt. Die prozentuale Hemmung der Nitrifikation in den behandelten Bodenproben wurde im Vergleich mit den unbehandelten Bodenproben (nur Ammoniumsulfat-Zusatz) nach folgender Formel berechnet:

$$\ldots \text{ \% Hemmung der Nitrifikation} = \frac{a-b}{a} \cdot 100$$

a = Nitrifikationsrate von Ammoniumsulfat (mit 100 % bzw. 1.0 angenommen)
b = Nitrifikationsrate von Ammoniumsulfat + Nitrifikationshemmer

| Wirkstoff Beispiel | ... % Hemmung der Nitrifikation 4 Wochen nach Zugabe von 1 ppm Wirkstoff zum Boden |
|---|---|
| 1 | 100 |
| 2 | 87 |
| 14 | 100 |
| 25 | 92 |
| 38 | 100 |
| 47 | 83 |
| 48 | 84 |
| 52 | 90 |
| 55 | 88 |
| 56 | 93 |
| 64 | 100 |
| 65 | 94 |
| 67 | 88 |
| 68 | 89 |
| 69 | 73 |
| 89 | 85 |
| 90 | 95 |
| 91 | 92 |
| 94 | 86 |
| 100 | 90 |
| 105 | 95 |
| 115 | 100 |
| 116 | 86 |
| 118 | 100 |
| 122 | 79 |
| 124 | 88 |
| 125 | 86 |
| 126 | 86 |
| 134 | 96 |
| 135 | 100 |
| 137 | 73 |
| 138 | 88 |
| 145 | 100 |
| 146 | 100 |
| 154 | 100 |
| 163 | 100 |

Vergleich

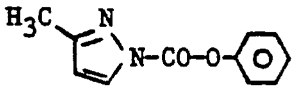 69

(Wirkstoff Nr. 95 der DE-OS 27 45 833)

**Patentansprüche**

1. Nitrifikationsinhibierende 1-Hydroxipyrazolderivate der Formel (I)

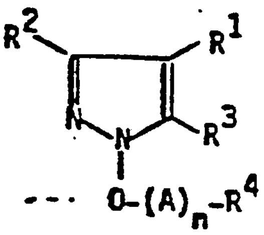 (I)

in der bedeuten:

A den Rest $-\overset{O}{\overset{\|}{C}}-$, $-\overset{O}{\overset{\|}{C}}-O-$, $-\overset{O}{\overset{\|}{C}}-\overset{H}{N}-$, -SO-, $-SO_2-$ oder $-SO_2-\overset{R^4}{N}-$,

n 0 und 1,

$R^1$, $R^2$ und $R^3$ unabhängig voneinander Wasserstoff, Chlor, Brom oder Iod,

$R^4$ Wasserstoff, einen verzweigten oder unverzweigten, offenkettigen oder einen cyclischen Alkyl-, Alkenyl- oder Alkinylrest oder einen Aryl- bzw. Aralkylrest mit der Maßgabe, daß $R^4$ nicht Wasserstoff ist, wenn n = 0 ist, und ferner mit der Maßgabe, daß die Verbindung N-Hydroxypyrazol-4-chlorbenzolsulfonat vom Schutz ausgenommen ist, wobei die substitutionsfähigen Reste $R^4$ auch ein- oder mehrfach durch Halogen, Halogenalkyl, Halogenalkoxi, Alkyl, Alkyloxi, Alkoxicarbonyl, -OH, =O, -COOH bzw. Salze davon, $-NO_2$ oder -CN substituiert sein können.

2. Nitrifikationsinhibierende Mittel, enthaltend in wirksamer Menge oder als Konzentrat wenigstens eine der Verbindungen der Formel I gemäß Anspruch 1, mit der Maßgabe, daß $R^4$ Wasserstoff sein kann, wenn n = 0 ist.

3. Verwendung eines Mittels gemäß Anspruch 2, zur Inhibierung der Nitrifikation durch Einwirkung auf den Boden.

4. Verwendung von N-Hydroxypyrazol-4-chlorbenzolsulfonat zur Inhibierung der Nitrifikation durch Einwurkung auf den Boden.

**Claims**

1. A nitrification-inhibiting 1-hydroxypyrazole derivative of the formula (I)

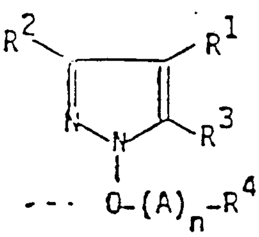 (I)

where A is $-\overset{O}{\overset{\|}{C}}-$, $-\overset{O}{\overset{\|}{C}}-O-$, $-\overset{O}{\overset{\|}{C}}-\overset{H}{N}-$, -SO- $-SO_2-$ or $-SO_2-\overset{R^4}{N}-$, n is 0 or 1, $R^1$, $R^2$ and $R^3$ independently of one another are each hydrogen, chlorine, bromine or iodine, and $R^4$ is hydrogen, a straight-chain or branched, open-chain alkyl, alkenyl or alkynyl radical, a cyclic alkyl, alkenyl or alkynyl radical or aryl or aralkyl, with the proviso that $R^4$ is not hydrogen when n is 0, and with the further proviso that the compound N-hydroxypyrazole-4-chlorobenzenesulfonate is excluded from protection, and the radicals $R^4$ which are capable of being substituted may furthermore be monosubstituted or polysubstituted by halogen, haloalkyl, haloalkoxy, alkyl, alkoxy, alkoxycarbonyl, -OH, =O, -COOH or salts of this, $-NO_2$ or -CN.

2. A nitrification-inhibiting agent, containing, in an effective amount or as a concentrate, one or more compounds of the formula I as claimed in claim 1, with the proviso that $R^4$ can be hydrogen when n is 0.

3. Use of an agent as claimed in claim 2 for inhibiting nitrification through action on the soil.

4. Use of N-hydroxypyrazole-4-chlorobenzenesulfonate for inhibiting nitrification through action on the soil.

**Revendications**

1. Dérivés de 1-hydroxypyrazol, inhibiteurs de nitrification, de formule (I)

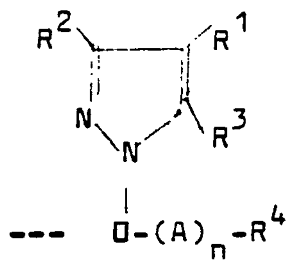

(I)

dans laquelle

A représente le reste $-\overset{O}{\overset{\|}{C}}-$, $-\overset{O}{\overset{\|}{C}}-O-$, $-\overset{O}{\overset{\|}{C}}-\overset{H}{N}-$, $-SO-$, $-SO_2-$ ou $-SO_2-\overset{R^4}{N}-$,

n représente 0 et 1

$R^1$, $R^2$ et $R^3$ indépendant l'un de l'autre, hydrogène, chlore, brome ou iode,

$R^4$, hydrogène, un reste alkyle, alcényle ou alcinyle, ramifié ou non ramifié, à chaîne ouverte ou cyclique ou un reste aryle ou aralkyle, sous réserve que $R^4$ n'est pas l'hydrogène, quand n = 0, et sous réserve encore qu'est exclu de la protection le composé sulfonate de N-hydroxypyrazol -4-chlorobenzène, le reste substituable $R^4$ pouvant être aussi substitué, une ou plusieurs fois par halogène, halogénoalkyle, halogénalcoxy, alkyle, alkyloxy, alcoxycarbonyle, -OH, =O, -COOH ou des sels de ceux-ci, $-NO_2$ ou -CN.

2. Agent inhibiteur de nitrification, contenant, en quantité efficace ou sous forme de concentré, au moins un des composés de formule I selon la revendication 1 sous réserve que $R^4$ peut être l'hydrogène lorsque n = 0.

3. Utilisation d'un agent selon la revendication 2 pour inhiber la nitrification par action sur le sol.

4. Utilisation de sulfonate de N-hydroxypyrazol-4-chlorobenzène pour inhiber la nitrification par action sur le sol.